# EUROPÄISCHE PATENTANMELDUNG

(11) **EP 3 028 652 A1**
(43) Veröffentlichungstag der Anmeldung: **08.06.2016**
(21) Anmeldenummer: 14004067.6
(22) Anmeldetag: 03.12.2014
(51) Int. Cl.: A61B 17/12, A61B 17/00, A61F 2/848, A61F 2/01, A61B 17/56

(54) **Verschlussvorrichtung geeignet zum Verschließen des linken Herz Ohrs**

(71) Anmelder: Peter Osypka Stiftung, 79639 Grenzach-Wyhlen (DE)
(72) Erfinder: Osypka, Peter, 79639 Grenzach-Wyhlen (DE)

(57) **Zusammenfassung**

Die vorliegende Anmeldung betrifft eine Verschlussvorrichtung, die insbesondere geeignet ist, das linke Herz Ohr zu verschließen. Die Verschlussvorrichtung besteht aus einem Geflecht (1) aus Formgedächtnismaterial, einer Zugfeder (2) und mindestens einem Fixierungselement (3). Geflecht (1) ist proximal scheibenförmig geformt, sodass eine Verschluss-Scheibe (4) vorhanden ist, die der Anatomie des Ostium im Ziel-Herz Ohr maßstabsgetreu entspricht. Geflecht (1) ist distal mit einer Zugfeder (3) verbunden, wobei die Zugfeder distal mindestens ein Fixierungselement aufweist.

## Beschreibung

Die vorliegende Erfindung betrifft eine Verschlussvorrichtung, die sich zum Verschließen des linken Herz Ohrs (Vorhofohr, left atrial appendage LAA, LAA-Occluder) eignet und ein Verfahren zu ihrer Herstellung.

Echokardiografische Studien haben gezeigt, dass Embolien die bei Menschen mit Vorhofflimmern für das erhöhte Schlaganfallrisiko verantwortlich sind, in mehr als 90 Prozent der Fälle vom linken Herz Ohr ausgehen. Das linke Herz Ohr ist eine Ausstülpung des linken Vorhofs und ein Überbleibsel aus der Embryonalentwicklung. Da das linke Herz Ohr keine Funktion zu haben scheint, liegt es nahe das linke Herz Ohr zu verschließen. (Deutsches Ärzteblatt vom 14.8.2009).

Wie hoch das Schlaganfallrisiko bei Vorhofflimmern ist, scheint auch durch die besondere anatomische Struktur des Herz Ohrs determiniert zu sein. (Ärzte Zeitung online, 01.08.2012). Man unterscheidet verschiedene anatomische Strukturen wie beispielsweise "Windsock Type, ChickenWing Type und Broccoli Type".

Wegen der komplexen Anatomie des linken Herz Ohrs und den vielfältigen Formen, die das Herz Ohr haben kann, ist ein Verschließen und Abdichten des linken Herz Ohrs durch einen LAA-Occluders eine besondere Herausforderung.

Das Europäische Patent EP2074953B1 beschreibt eine Verschlussvorrichtung bestehend aus zwei selbstexpandierenden Nitinolschirmen, wobei der Nitinolschirm, der sich nach der Implantation innerhalb des Herz Ohrs befindet mehrere Haken aufweist, die sich aus dem Nitinolschirm heraus erstrecken und konfiguriert sind sich in der Innenwand des Herz Ohrs zu verankern. Die Haken bestehen aus einzelnen Drähten innerhalb des Nitinolschirmgeflechts. Die Nitinolschirme werden in zusammengefalteter Form mit Hilfe eines Einführungskatheters zum Herz Ohr geführt. Sobald die Nitinolschirme aus dem Einführungskatheter geschoben werden entfalten sie sich.

Aufgabe der vorliegenden Erfindung ist es eine Verschlussvorrichtung zu entwickeln, die in ihrer Form individuell an die Form des Herz Ohrs eines jeden Patienten angepasst ist; die sicher eingeführt werden kann, nach der Einführung dicht und zuverlässig fixiert ist und sich nicht aus ihrer Verankerung lösen kann.

Die Aufgabe wird gelöst durch ein Geflecht aus Formgedächtnismaterial, wobei das Geflecht Patienten spezifisch geformt ist. Das Geflecht ist proximal scheibenförmig geformt, wobei die Verschluss-Scheibe der Anatomie des Ostium im Ziel-Herz Ohr maßstabsgetreu entspricht und somit in den Eingang (Ostium) des Herz Ohrs exakt eingepasst werden kann. Die zuverlässige Fixierung des Geflechts wird über eine Zugfeder mit distal angeordneten Fixierungselementen erreicht.

Die vorliegende Erfindung betrifft eine Patienten spezifisch geformte Verschlussvorrichtung geeignet zum Verschließen des linken Herz Ohrs, wobei die Verschlussvorrichtung aus einem Geflecht (1) aus Formgedächtnismaterial, einer Zugfeder (2) und mindestens einem Fixierungselement (3) besteht, dadurch gekennzeichnet, dass das Geflecht (1) proximal scheibenförmig geformt ist, sodass proximal eine Verschluss-Scheibe (4) vorhanden ist, deren Form der Anatomie des Ostium im Ziel-Herz Ohr maßstabsgetreu entspricht und wobei Geflecht (1) distal mit einer Zugfeder (2) verbunden ist, die distal mindestens ein Fixierungselement (3) aufweist.

Der Ausdruck "Geflecht aus Formgedächtnismaterial" umfasst alle Materialien die nach einer starken Verformung ihre frühere Formgebung wiedererlangen können wie Formgedächtnislegierungen (z. B. Nitinol) und Formgedächtnispolymere (biokompatible Kunststoffgeflechte, absorbierbare Kunststoffe).

Die Patienten spezifische Formgebung des Geflechts geschieht auf Basis von digitalen Aufnahmen des Patientenherzes/Herz Ohrs. Die digitalen Aufnahmen können aus medizinischen Aufnahmen wie beispielsweise MRT-, CT-, Ultraschall-, oder Röntgenaufnahmen unter Verwendung von handelsüblicher CAD-und/oder Bildgebungssoftware rekonstruiert werden. Dabei wird ein dreidimensionaler Bilddatensatz rekonstruiert, der das Herz Ohr maßstabsgetreu darstellt. Vorteilhafterweise wird die geometrische Information über das Herz Ohr in einem dem DICOM Standard (Digital Imaging and Communication in Medicine) entsprechenden Format gespeichert. Aus dem 3D-DICOM Datensatz wird nach Datensegmentierung ein 3D-Objekt gespeichert, welches in ein Format umgewandelt wird, das sich zur Herstellung eines 3D-Modells eignet. Vorzugsweise wird ein STL Format verwendet. Aus dem STL Format wird ein Modell des Herz Ohrs gewonnen, indem ausgehend vom Computer generierten Modell ein entsprechendes reales Modell gefertigt wird. Die Daten werden beispielsweise an einen 3-D Drucker übermittelt und das Modell des Herz Ohrs wird durch selektives Laser-Sintern hergestellt. Das so hergestellte Modell dient zur Formgebung des Geflechts sowie zur Herstellung der erfindungsgemäßen Verschlussvorrichtung.

Das Modell kann aus allen temperaturbeständigen Werkstoffen bestehen, die zum Laser-Sintern geeignet sind. In einer Ausführungsform ist das Modell aus Stahl.

In einer Ausführungsform ist das Geflecht ein Metallgeflecht aus Nitinol. Das Metallgeflecht ist somit selbstexpandierend und besitzt eine erste zusammengefaltete Konfiguration und eine zweite expandierte Konfiguration.

Das Metallgeflecht besteht beispielsweise aus mindestens 30 Nitinolfäden, vorzugsweise 30-100 Fäden mit einem Durchmesser von 0,03-0,15mmm.

Geflecht (1) hat im entfalteten Zustand die Form des Eingangsbereichs des Herz Ohrs und ist vorgesehen das Ostium (Eingangsöffnung) des Herz Ohrs zu verschließen. Das Geflecht ist ein Hohlkörper mit einer geflochtenen Wandung. Proximal ist das Geflecht scheibenförmig ausgebildet, sodass eine Verschlussscheibe (4) in einen in etwa zylinderförmigen oder kegelförmigen Geflechts-Körper mündet, dessen Ende durch eine Hülse (5) gehalten ist. Die Außenkontur der Verschlussscheibe entspricht der Geometrie der Herzohröffnung.

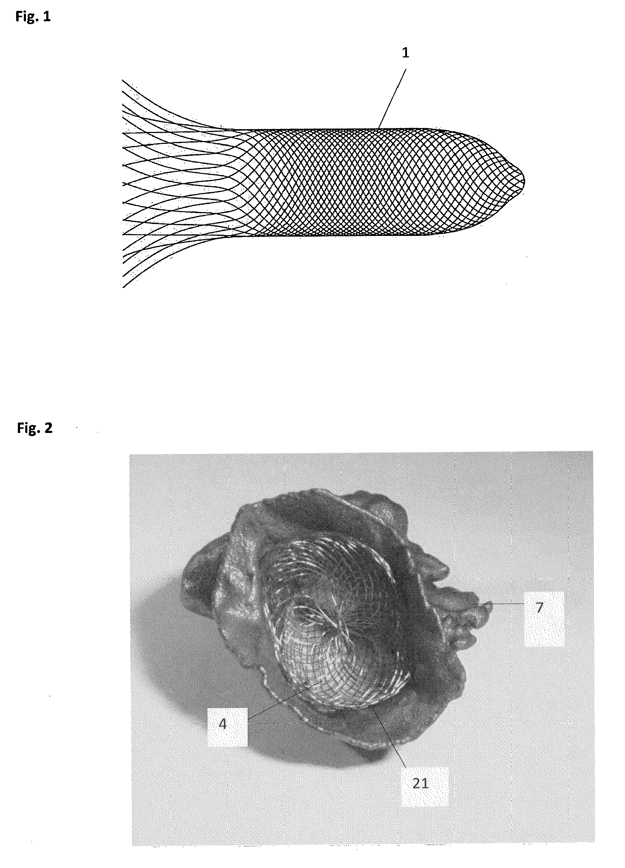

Wie Fig. 1 zeigt wird zunächst ein schlauchförmiges Netz geflochten, dessen Enden gefasst werden.

Fig. 2 zeigt den wesentlichen erfindungsgemäßen Vorgang. Das schlauchförmige Netz wir in die durch Laser-sintern hergestellte Form gepresst, die dem Herz Ohr des Patienten entspricht. Das Netz verformt sich zur Verschlussscheibe, wobei zwei unterschiedliche Netzbereiche scheibenförmig aufeinanderliegen. So entsteht ein Doppelgeflecht. Es ist aber auch möglich die Verschlussscheibe als Einfachgeflecht zu gestalten. Fig. 2 zeigt sehr gut den entscheidenden Vorteil, den das Modell des Herz Ohrs bietet. Wäre die Verschluss-Scheibe z. B. kreisförmig, könnte die Verschluss-Scheibe das Ostium des Herz Ohrs nicht dicht verschließen..

Das Geflecht kann eine dünne Kunststoffmembran (Silikon, Polyurethan, PTEE) aufweisen, die ganz oder teilweise das Geflecht auf der Außenseite oder auf der Innenseite bedeckt. Dies führt zu einer Verbesserung der Dichtigkeit.

An die Hülse (5) schließt sich distal die Zugfeder und das Fixierungselement bzw. die Fixierungselemente an.

Die Zugfeder besteht aus einem hochelastischen Material. Geeignet ist jedes biokompatible Material welches sich elastisch rückstellend verhält. Die Zugfeder sorgt für eine hinreichende Anpresskraft von Geflecht (1) an die Eingangsöffnung des Herz Ohrs und somit für einen zuverlässigen Verschluss.

In einer Ausführungsform ist die Zugfeder eine Wendel, die aus einem rohrförmigen Materialstück aus Formgedächtnismaterial, vorzugsweise aus Nitinol ausgeschnitten wird. Die Länge der Wendel ist von den anatomischen Gegebenheiten des Herz Ohrs abhängig.

An die Zugfeder schließt distal das Fixierungselement an. Auch das Fixierungselement wird wie die Zugfeder vorteilhafterweise aus dem rohrförmigen Materialstück ausgeschnitten aus dem auch die Zugfeder ausgeschnitten wurde. In einer Ausführungsform besteht das Fixierungselement aus einem Nitinoldraht. In einer bevorzugten Ausführungsform sind mehrere Nitinoldrähte vorhanden, die sich radial um die virtuell verlängerte Mittelachse der Zugfeder gruppieren. Sie gruppieren sich vorzugsweise in gleichmäßigem Abstand. Sind beispielsweise 4 Nitinoldrähte vorhanden, beträgt der Abstand circa 90 Grad. Sind beispielsweise 3 Nitinoldrähte vorhanden, beträgt der Abstand circa 120 Grad. Sind beispielsweise 10 Nitinoldrähte vorhanden, so beträgt der Abstand circa 36 Grad.

Der Ausdruck "mehrere Nitinoldrähte" beinhaltet mindestens zwei Drähte oder Streifen, vorzugsweise drei bis vier Drähte (Streifen). In einer Ausführungsform sind 4-10 Drähte vorhanden, besonders bevorzugt sind vier Drähte.

Die Fixierungselemente bestehen aus Drähten aus Formgedächtnismaterial. Jeder Draht ist an seinem distalen Ende nach außen hakenförmig gebogen. Nach außen gebogen meint eine Biegung zur Innenwand des Herz Ohrs hin. Das distale Ende des Fixierungselements (Haken) muss so geformt sein, dass eine Verankerung in der Innenwand des Herz Ohrs möglich ist, das Fixierungselement aber nicht durch die Herzohrwand hindurchtreten kann. Die Krümmung des Hakens folgt im Wesentlichen einem Halbkreis oder einer V-Form.

Die Länge der zur Fixierung dienenden Drähte kann gleich sein, vorzugsweise variiert jedoch die Länge der Fixierungselemente um die unterschiedlichen anatomischen Gegebenheiten des Herz Ohrs zu berücksichtigen. Die Fixierungselemente müssen jedoch so lang sein, dass sie in Gebrauchsstellung die Innenwand des Herz Ohrs erreichen, um sich dort zu verankern.

Vorzugsweise besteht das Fixierungselement aus demselben Formgedächtnismaterial wie Zugfeder und Geflecht.

Die Länge und Ausgestaltung von Zugfeder und Fixierungselementen werden auch auf Basis des Herzohrmodells festgelegt.

Obwohl die Fertigung der Zugfeder mit dem anschließenden Fixierungselement (den anschließenden Fixierungselementen) aus ein und demselben Material am einfachsten ist, sind auch andere Kombinationen denkbar. So könnte sich beispielsweise an das Metallgeflecht eine Zugfeder aus einem elastischen Kunststoff anschließen. Als Fixierungselement kämen auch andere bekannte Elemente in Frage wie loop, t-bar-förmiger Anker, Schraube oder Haken.

Geflecht (1) kann proximal eine Öffnung aufweisen, sodass gemeinsam mit der Zugfeder ein zentrales Lumen (6) entsteht, welches mit einem selbstdichtenden Ventil abgedichtet wird. Ein zentrales Lumen ist von Vorteil, wenn beispielsweise ein Stilett, ein Führungsdraht (guide wire) oder ein Führungskatheter hindurchgeschoben werden soll.

Selbstdichtende Ventile sind z. B. Druckventile wie z.B. Silikonventile, die im Handel in vielen Formen und Größen erhältlich sind. Beispielsweise können geeignete Ventile bei Firma Minivalve Int. B.V. bezogen werden.

Die vorliegende Erfindung betrifft auch ein Verfahren zur Herstellung eines LAA Occluders.

Das Verfahren umfasst die folgenden Schritte:
a) Bereitstellung eines Computer generierten 3-D Modells des Herz Ohrs auf der Basis von medizinischen Aufnahmen des Herz Ohrs,
b) Übermittlung der Daten des Computer generierten Modells an einen 3-D Drucker,
c) Herstellung eines realen Modells (7) durch selektives Laser-Sintern;
d) Herstellung eines Geflechts aus Formgedächtnismaterial und Formgebung des Geflechts durch Pressen des Geflechts in das in Schritt c) erhaltene Modell oder ein Folgemodell wobei durch das Pressen Geflecht (1) entsteht welches proximal scheibenförmig geformt wird, sodass eine Verschluss-Scheibe (4) entsteht, deren Form der Anatomie des Ostium im Ziel-Herz Ohr maßstabsgetreu entspricht;
e) tempern des Geflechts (1) zur Fixierung der Formänderung,
f) Fassung der Geflechts-Enden in einer Hülse (5) und Verbindung des Geflechts mit der Zugfeder (2), die distal mindestens ein Fixierungselement (3) aufweist.

Ist das Geflecht ein Nitinol-Geflecht findet das Tempern bei 400-600 Grad C statt.

Der Ausdruck medizinische Daten umfasst MRT-, CT-, Ultraschall- oder Röntgenaufnahmen.

Unter dem Begriff Folgemodell wird eine Form verstanden, die sich aus dem in Schritt c) erhaltenen Modell direkt ableitet beispielsweise durch ausschneiden von Teilbereichen.

Die vorliegende Erfindung betrifft somit auch eine Verschlussvorrichtung hergestellt nach dem oben beschriebenen Verfahren.

Wesentlich ist Verfahrensschritt a-e, wobei ein Geflecht aus Formgedächtnismaterial entsteht das proximal scheibenförmig geformt ist, sodass eine Verschluss-Scheibe vorhanden ist, deren Form der Anatomie des Ostium im Ziel-Herz Ohr maßstabsgetreu entspricht.

Die erfindungsgemäße Verschlussvorrichtung wird mit Hilfe eines Einführungskatheters eingeführt.

Als Einführungskatheter können gebräuchliche Einführungskatheter verwendet werden. Eine besonders geeignete Vorrichtung ist beispielsweise in EP 2090230 beschrieben. Die in EP2090230 beschriebene Vorrichtung dient zum Implantieren eines Verschlusses im Septum der Vorhöfe und weist einen Katheter mit einem in dessen Längsrichtung im Inneren bewegbaren Schiebeelement (pusher) auf.

Mit Hilfe des Einführungskatheters wird das zusammengefaltete Geflecht zum linken Herz Ohr geführt. Auch die Fixierungselemente und die Zugfeder befinden sich im Einführungskatheter. Wenn der Katheter sein Zielgebiet erreicht hat, wird der Verschluss mit Hilfe des Schiebeelements (Pusher) aus dem Einführungskatheter geschoben. Zuerst treten die Fixierungselemente aus. Die Fixierungselemente sind so getempert, dass sie sich bei Verlassen des Einführungskatheters entfalten und sich radial um die virtuell verlängerte Mittelachse der Zugfeder in Längsrichtung erstrecken und sich nach außen zur Innenwand des Herz Ohrs hin neigen. Wenn die Fixierungselemente die Innenwand des Herz Ohrs (die wie oben ausgeführt sehr zerklüftet sein kann) erreichen, verankern sie sich und das Geflecht wird fixiert. Das Verankern erfolgt durch leichten Zug am Schiebeelement.

Für eine verbesserte Führung der erfindungsgemäßen Verschlussvorrichtung kann ein Stilett, ein Führungskatheter, ein Führungsdraht (guide wire) durch das zentrale Lumen geschoben werden, an dessen distalem Ende sich ein Verankerungselement befindet, eine Schraube, ein T-bar oder ein Anker. Der Führungskatheter wird in Gebrauchsstellung in der hinteren Innenwand des Herz Ohrs fixiert.

Das Geflecht wird mit Hilfe des Schiebeelements aus dem Einführungskatheter hinausgeschoben. Dabei entfaltet sich Geflecht und nimmt seine zuvor durch Tempern bestimmte Patienten spezifische-Form an.

Der Einführungskatheter wird zurückgezogen.

Geflecht (1) ist bei Einführung im proximalen Bereich mit einem Faden versehen. Dieser Faden unterstützt eine eventuell notwendige Repositionierung.

Nachstehend ist ein Ausführungsbeispiel der Erfindung anhand der Zeichnung beschrieben.

Die Bezifferung der Figuren ist:
- 1: Geflecht
- 2: Zugfeder
- 3: Fixierungselement
- 4: Verschluss-Scheibe
- 5: Hülse
- 6: Lumen
- 7: Modell Herz Ohr des Patienten
- 8: Ausstülpung des Herz Ohrs
- 9: Ventil
- 10: Einführungskatheter
- 11: pusher
- 12: Faden
- 20: Herz Ohr
- 21: Ostium
- 22: Herz Ohr Innenwand

**Fig. 1** zeigt die Entstehung des Geflechts (1), hier ein Metallgeflecht aus Nitinol. Zunächst wird ein Hohlkörper geflochten. Der Hohlkörper weist eine Längsachse und eine Wandung auf.
**Fig. 2** zeigt die Formgebung des geflochtenen Hohlkörpers aus Fig. 1. Das Geflecht wird in das aus Metall bestehende reale Modell (7) des Herz Ohrs gepresst, wobei das Patienten spezifisch geformte Geflecht mit Verschluss-Scheibe (4) entsteht, welches anschließend getempert wird. Das Modell (7) wurde durch Laser-sintern hergestellt. Man erkennt gut wie sich das Geflecht (1) in den Herz Ohr Eingang (21) einfügt und dieses dicht verschließt.

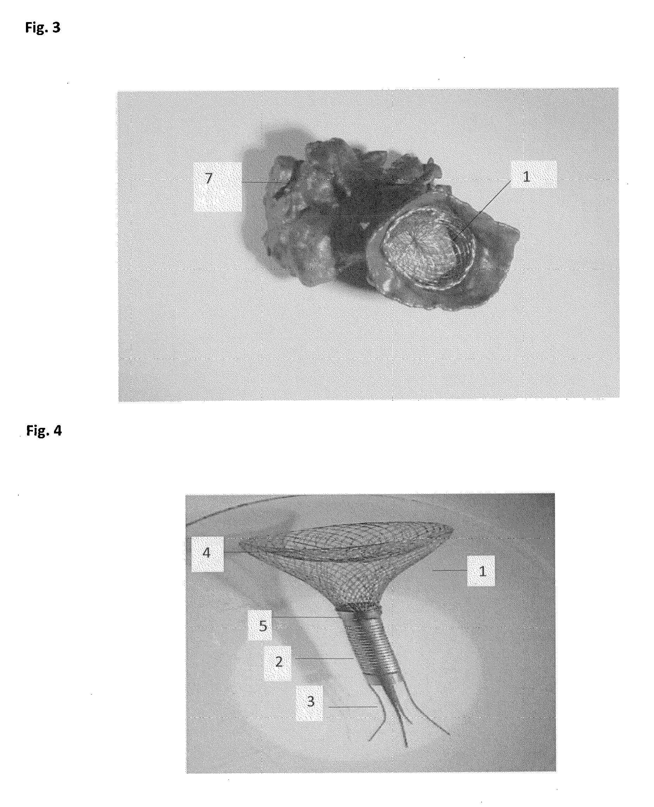

**Fig. 3** zeigt das Modell (7) in Seitenansicht. Man erkennt wie zerklüftet die Oberfläche des Herz Ohrs sein kann. Geflecht (1) ist eingepresst.
**Fig. 4** zeigt die Verschlussvorrichtung. Man erkennt das Metallgeflecht (1) welches proximal scheibenförmig geformt ist, sodass eine Verschluss-Scheibe (4) entsteht, die als Doppelscheibe ausgebildet ist. Metallgeflecht (1) mündet in die Halterung (5). Daran schließt sich distal die Zugfeder (2) an, die vier Fixierungselemente (3) aufweist, die sich radial um den distalen Rand der Zugfeder gruppieren. Die Zugfeder ist eine Metallwendel, die aus einem Metallrohr ausgeschnitten wurde. Die Fixierungselemente wurden ebenfalls aus dem Metallrohr ausgeschnitten.

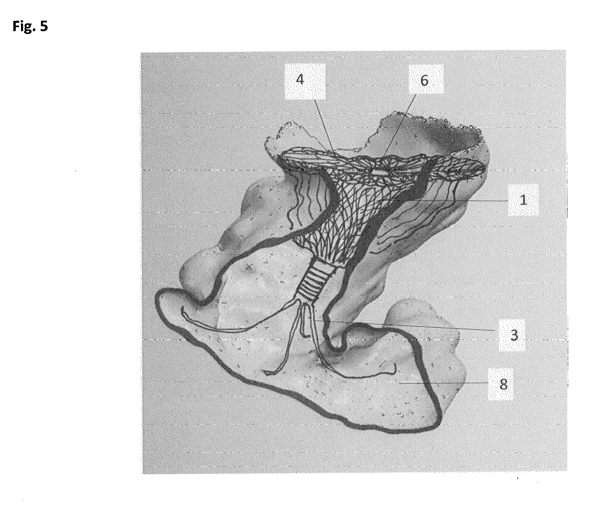

**Fig. 5** zeigt die einsatzbereite fertiggestellte Verschlussvorrichtung. Man erkennt wie sich die Verschlussvorrichtung in das Herz Ohr passgenau einfügt. Wie eingangs erwähnt kann das Herz Ohr sehr viele verschiedene Formen haben. Fig. 5 zeigt in Modellform ein Herz Ohr mit einer Ausstülpung (8). Um dieser speziellen Anatomie Rechnung zu tragen ist eines der Fixierungselemente (3) länger, damit eine Verankerung in der Ausstülpung gewährleistet ist. Geflecht (1) weist proximal eine Öffnung auf, sodass gemeinsam mit der Zugfeder ein zentrales Lumen (6) entsteht.

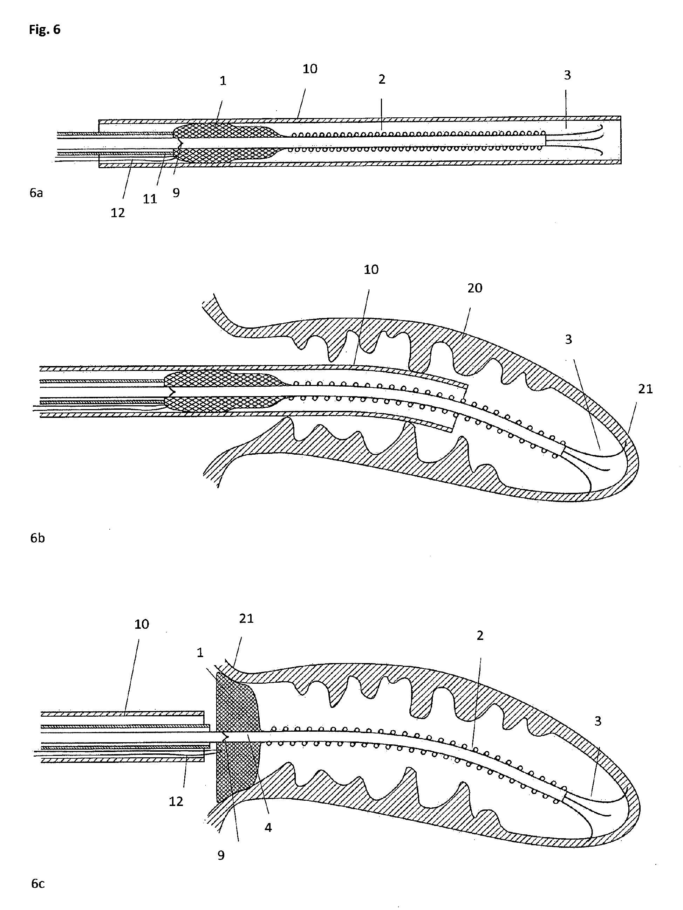

**Fig. 6** zeigt schematisch die Einführungsvorrichtung für das erfindungsgemäße Verschlusssystem und den Einführungs- und Implantierungsvorgang.
**Fig. 6a** zeigt die Einführungsvorrichtung in Ausgangsposition. Zu sehen ist der Einführungskatheter (10) im Querschnitt mit dem zusammengefalteten Geflecht (1), die Zugfeder (2) und die zusammengepressten Fixierungselemente (3). Der Einführungskatheter ist distal offen, sodass die Verschlussvorrichtung mit Hilfe eines Pushers (11) herausgeschoben werden kann. Die nach außen geformten Nitinol Fixierungselemente (3) befinden sich am distalen Rand der Zugfeder und erstrecken sich in Längsrichtung. Druckventil (9) befindet sich im proximalen Teil von Metallgeflecht (1). Ein Pusher (11) drückt über das Metallgeflecht (1) die gesamte Verschlussvorrichtung aus dem Einführungskatheter. Faden (12) dient einer eventuellen Repositionierung.
**Fig. 6b** zeigt den in das linke Herz Ohr (20) eingeschobenen Einführungskatheter (10). Die Fixierungselemente (3) wurden herausgeschoben und haben sich radial entfaltet. Aufgrund ihres hakenförmigen Endes haben sich die Fixierungselemente in der Innenwand (21) des Herz-Ohrs verankert. Die Verschlussvorrichtung ist jetzt fixiert. Die Zugfeder (2) tritt hervor.
**Fig. 6c** zeigt den Fortgang der Implantierung. Der Einführungskatheter (10) ist weiter zurückgezogen worden. Metallgeflecht (1) hat sich entfaltet. Lumen (4) wird durch Ventil (9) verschlossen. Aufgrund der Verankerung durch die Fixierungselemente (3) wird die Zugfeder (2) bei Rückzug von Metallgeflecht (1) gespannt. Nach Entspannung der Zugfeder fügt sich Metallgeflecht (1) in das Ostium (21) ein und schließt dieses dicht ab. Falls gewünscht kann eine Repositionierung erfolgen. Bei Repositionierung kann die Verschlussvorrichtung mit Hilfe von Faden (12) herausgezogen und nach Wunsch gedreht werden. Der faden (12) ist ein Doppelfaden, der nach erfolgter Implantation entfernt wird.

## Patentansprüche

1. Verschlussvorrichtung geeignet zum Verschließen des linken Herz Ohrs, wobei die Verschlussvorrichtung aus einem Geflecht (1) aus Formgedächtnismaterial, einer Zugfeder (2) und mindestens einem Fixierungselement (3) besteht, **dadurch gekennzeichnet, dass** das Geflecht (1) proximal scheibenförmig geformt ist, sodass proximal eine Verschluss-Scheibe (4) vorhanden ist, deren Form der Anatomie des Ostium im Ziel-Herz Ohr maßstabsgetreu entspricht und wobei Geflecht (1) distal mit einer Zugfeder (2) verbunden ist, die distal mindestens ein Fixierungselement (3) aufweist.

2. Verschlussvorrichtung nach Anspruch 1, wobei das Geflecht aus Nitinol besteht.

3. Verschlussvorrichtung nach Anspruch 1 oder 2, wobei das Geflecht (1) mit einer Kunststoffmembran ganz oder teilsweise auf der Außenseite oder auf der Innenseite bedeckt ist.

4. Verschlussvorrichtung nach Anspruch 1, wobei die Zugfeder aus Nitinol besteht.

5. Verschlussvorrichtung nach Anspruch 1, wobei das Fixierungselement aus Nitinoldraht besteht welches am distalen Ende hakenförmig gebogen ist..

6. Verschlussvorrichtung nach Anspruch 1 oder 5, wobei 3-10 Fixierungselemente vorhanden sind, die sich radial um die virtuell verlängerte Mittelachse der Zugfeder gruppieren.

7. Verschlussvorrichtung nach Anspruch 6, wobei 4 Nitinoldrähte vorhanden sind, die einen Abstand von circa 90 Grad zueinander aufweisen.

8. Verschlussvorrichtung nach Anspruch 1-7, wobei die Verschlussvorrichtung ein zentrales Lumen (4) und ein selbstdichtendes Ventil (9) aufweist, welches Lumen (4) nach außen abdichtet.

9. Verfahren zur Herstellung einer Verschlussvorrichtung, die geeignet ist das linke Herz Ohr zu verschließen, wobei das Verfahren die folgenden Schritte umfasst:
a) Bereitstellung eines Computer generierten 3-D Modells des Herz Ohrs auf der Basis von medizinischen Aufnahmen des Herz Ohrs,
b) Übermittlung der Daten des Computer generierten Modells an einen 3-D Drucker,
c) Herstellung eines realen Modells (7) durch selektives Laser-Sintern;
d) Herstellung eines Geflechts aus Formgedächtnismaterial und Formgebung des Geflechts durch Pressen des Geflechts in das in Schritt c) erhaltene Modell oder ein Folgemodell wobei durch das Pressen Geflecht (1) entsteht welches proximal scheibenförmig geformt wird, sodass eine Verschluss-Scheibe (4) entsteht, deren Form der Anatomie des Ostium im Ziel-Herz Ohr maßstabsgetreu entspricht;
e) tempern des Geflechts (1) zur Fixierung der Formänderung,
f) Fassung der Geflechts-Enden in einer Hülse (5) und Verbindung des Geflechts mit der Zugfeder (2), die distal mindestens ein Fixierungselement (3) aufweist.

10. Verschlussvorrichtung gemäß Anspruch 1 hergestellt nach einem Verfahren gemäß Anspruch 9.

11. Geflecht (1) aus Formgedächtnismaterial das proximal scheibenförmig geformt ist, sodass eine Verschluss-Scheibe (4) vorhanden ist, deren Form der Anatomie des Ostium im Ziel-Herz Ohr maßstabsgetreu entspricht.

12. Einführungskatheter bestehend aus einem Einführungskatheter (10) in den die Verschlussvorrichtung gemäß einem der Ansprüche 1-8 in zusammengefalteter Konfiguration eingeführt ist.
